# EUROPÄISCHE PATENTSCHRIFT

(11) **EP 4 563 083 B1**
(45) Veröffentlichungstag und Bekanntmachung des Hinweises auf die Patenterteilung: **25.02.2026**
(21) Anmeldenummer: 23213237.3
(22) Anmeldetag: 30.11.2023
(51) Int. Cl.: A61B 5/055, A61B 5/00

(54) **MAGNETRESONANZVORRICHTUNG MIT EINER LÖSBAREN ANORDNUNG EINER FÜHRUNGSSCHIENENEINHEIT**
MAGNETIC RESONANCE DEVICE WITH A DETACHABLE ARRANGEMENT OF A GUIDE RAIL UNIT
DISPOSITIF DE RÉSONANCE MAGNÉTIQUE DOTÉ D'UN AGENCEMENT AMOVIBLE D'UNE UNITÉ DE RAIL DE GUIDAGE

(43) Veröffentlichungstag der Anmeldung: 04.06.2025
(73) Patentinhaber: Siemens Healthineers AG, 91301 Forchheim (DE)
(72) Erfinder: HETZ, Christian, 91362 Pretzfeld (DE); KURTH, Rainer, 91056 Erlangen (DE); SCHRAMM, Martin, 90542 Eckental (DE); ZINK, Stephan, 90451 Nürnberg (DE)
(74) Vertreter: Siemens Healthineers Patent Attorneys

(56) Entgegenhaltungen:
- EP-A1- 4 014 855
- CN-A- 110 722 864
- DE-U1- 202022 105 292
- US-A1- 2016 135 896
- US-A1- 2022 022 829

## Beschreibung

Die vorliegende Erfindung betrifft eine Magnetresonanzvorrichtung mit einer Scannereinheit, einen von der Scannereinheit zumindest teilweise umgebenen Patientenaufnahmebereich, wobei die Scannereinheit eine den Patientenaufnahmebereich zumindest teilweise umgebende Umhausung aufweist, einer Patientenlagerungsvorrichtung mit einem bewegbaren Patiententisch, wobei der Patiententisch in den Patientenaufnahmebereich einfahrbar ausgebildet ist.

Unabhängig vom grammatikalischen Geschlecht eines bestimmten Begriffes sind Personen mit männlicher, weiblicher oder anderer Geschlechteridentität mit umfasst.

Magnetresonanzvorrichtung weisen für gewöhnlich einen Patientenaufnahmebereich auf, in dem ein Patient für eine Untersuchung, insbesondere eine Magnetresonanzuntersuchung, gelagert und/oder positioniert wird. Bevorzugt soll hierbei der Patient, insbesondere der zu untersuchende Bereich des Patienten, in einem Isozentrum der Magnetresonanzvorrichtung positioniert werden, um ideale Bedingungen für eine Erfassung von Magnetresonanzdaten zu erhalten. Hierzu ist der Patient auf einem Patiententisch einer Patientenlagerungsvorrichtung positioniert. Für eine horizontale Positionierung und/oder eine Positionierung in z-Richtung der Magnetresonanzvorrichtung wird der Patiententisch innerhalb des Patientenaufnahmebereichs so lange in z-Richtung verschoben und/oder verfahren, bis der zu untersuchende Bereich des Patienten im Isozentrum positioniert ist.

Insbesondere bei zylinderförmig ausgebildeten Patientenaufnahmebereichen von Magnetresonanzvorrichtungen weist der Patiententisch innerhalb des Patientenaufnahmebereichs in vertikaler Richtung eine einzige Position auf. Diese Position in vertikaler Richtung ist durch Führungsschienen, die an einer den Patientenaufnahmebereich umgebenden Umhausung angeordnet sind, festgelegt. Diese Position ermöglicht für einen Durchschnittspatienten eine im Wesentlichen optimale Positionierung innerhalb des Patientenaufnahmebereichs, insbesondere hinsichtlich des Isozentrums in vertikaler Richtung. Jedoch stellt dies Position in vertikaler Richtung für Patienten, die aufgrund ihrer Anatomie stark von einem durchschnittlichen Patienten abweichen, einen Kompromiss in der Positionierung dar. Insbesondere bei Kleinkindern und/oder Säuglingen oder stark adipös veranlagte Patienten ist eine Positionierung im Isozentrum in vertikaler Richtung sehr schwierig oder unmöglich, da Position des Patiententisch in vertikaler Richtung auch nicht verstellbar und/oder einstellbar ausgebildet ist. Dies führt dazu, dass unabhängig von einer Patientenanatomie und/oder einer Art der Magnetresonanzuntersuchung der Patiententisch in vertikaler Richtung immer die gleiche Position einnimmt.

Für eine Anpassung einer Position des Patienten in vertikaler Richtung ist es bisher erforderlich, den Patienten mit zusätzlichen Positionierhilfen, beispielsweise Positionierkissen, in eine gewünschte Position zu bringen. Eine derartige Positionierung ist jedoch zeitaufwendig für ein medizinisches Bedienpersonal. Jedoch ist hierbei nur eine Anpassung nach oben möglich. Eine Absenkung des Patiententischs innerhalb des Patientenaufnahmebereichs ist nicht möglich.

EP 4 014 855 A1 offenbart eine Magnetresonanzvorrichtung mit einer Vertikaleinstelleinheit zu einer Einstellung einer Vertikalposition des Patiententischs innerhalb eines Patientenaufnahmebereichs der Magnetresonanzvorrichtung.

DE 20 2022 105 292 A1 beschreibt eine Magnetresonanzvorrichtung mit einer Führungseinheit zu einer Führung eines Patiententischs innerhalb eines Patientenaufnahmebereichs der Magnetresonanzvorrichtung.

Der vorliegenden Erfindung liegt insbesondere die Aufgabe zugrunde, eine flexible Positionierung eines Patiententisch innerhalb des Patientenaufnahmebereichs zu ermöglichen. Die Aufgabe wird durch die Merkmale der unabhängigen Ansprüche gelöst. Vorteilhafte Ausgestaltungen sind in den Unteransprüchen beschrieben.

Die Erfindung geht aus von einer Magnetresonanzvorrichtung mit einer Scannereinheit, einen von der Scannereinheit zumindest teilweise umgebenen Patientenaufnahmebereich, wobei die Scannereinheit eine den Patientenaufnahmebereich zumindest teilweise umgebende Umhausung aufweist, einer Patientenlagerungsvorrichtung mit einem bewegbaren Patiententisch, wobei der Patiententisch in den Patientenaufnahmebereich einfahrbar ausgebildet ist. Erfindungsgemäß weist die Magnetresonanzvorrichtung zumindest zwei unterschiedliche Führungsschieneneinheiten auf, wobei die zumindest zwei unterschiedlichen Führungsschieneneinheiten unterschiedliche Führungshöhen für eine Führung des Patiententischs innerhalb des Patientenaufnahmebereichs aufweisen und wobei die zumindest zwei unterschiedlichen Führungsschieneneinheiten an der Umhausung auswechselbar positionierbar sind.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Die Magnetresonanzvorrichtung umfasst hierzu die Scannereinheit. Die Scannereinheit der Magnetresonanzvorrichtung umfasst bevorzugt eine Magneteinheit zur Erfassung der medizinischen und/oder diagnostischen Bilddaten. Bevorzugt umfasst hierbei die Scannereinheit, insbesondere die Magneteinheit, einen Grundmagneten, ein Gradientenspuleneinheit und eine Hochfrequenzantenneneinheit. Die Hochfrequenzantenneneinheit ist fest innerhalb der Scannereinheit angeordnet und zur Aussendung eines Anregungspulses ausgelegt und/oder ausgebildet.

Des Weiteren weist die Magnetresonanzvorrichtung zumindest eine lokalen Hochfrequenzspule auf, die zu einem Empfangen eines Magnetresonanzsignals ausgebildet ist. Hierzu wird die lokale Hochfrequenzspule um den zu untersuchenden Bereich des Patienten angeordnet und/oder angelegt. Bevorzugt sind die einzelnen lokalen Hochfrequenzspulen speziell auf einen Untersuchungsbereich von Patienten ausgelegt und/oder ausgebildet, wie beispielsweise eine Kopfhochfrequenzspule oder eine Kniehochfrequenzspule usw.

Der Grundmagnet ist zur Erzeugung eines homogenen Grundmagnetfelds mit einer definierten Magnetfeldstärke, wie beispielsweise mit einer Magnetfeldstärke von 3 T oder 1,5 T usw., ausgebildet. Insbesondere ist der Grundmagnet zur Erzeugung eines starken und konstanten Grundmagnetfelds ausgebildet. Das homogene Grundmagnetfeld ist bevorzugt innerhalb des Patientenaufnahmebereichs der Magnetresonanzvorrichtung angeordnet und/oder vorzufinden. Die Gradientenspuleneinheit ist zu einer Erzeugung von Magnetfeldgradienten, die für eine Ortskodierung während einer Bildgebung verwendet werden, ausgebildet.

Der Patientenaufnahmebereich ist zu einer Aufnahme des Patienten, insbesondere des zu untersuchenden Bereichs des Patienten, für eine medizinische Magnetresonanzuntersuchung ausgelegt und/oder ausgebildet. Der Patientenaufnahmebereich umfasst bevorzugt den Bereich, der dem Patienten während einer Magnetresonanzuntersuchung zur Verfügung steht. Beispielsweise ist hierzu der Patientenaufnahmebereich zylinderförmig ausgebildet und/oder zylinderförmig von der Scannereinheit, insbesondere der Magneteinheit, der Magnetresonanzvorrichtung umgeben. Insbesondere umfasst hierbei die Scannereinheit, insbesondere die Magneteinheit, eine den Patientenaufnahmebereich zumindest teilweise umgebende Umhausung. Bevorzugt umgibt dabei die Umhausung den Patientenaufnahmebereich zylinderförmig. Die Umhausung kann dabei einstückig mit der Hochfrequenzantenneneinheit der Magneteinheit ausgebildet sein und eine dem Patientenaufnahmebereich zugewandte Seite der Hochfrequenzantenneneinheit umfassen.

Innerhalb des Patientenaufnahmebereichs ist bevorzugt ein Field of View (FOV) und/oder ein Isozentrum der Magnetresonanzvorrichtung angeordnet. Das FOV umfasst bevorzugt einen Erfassungsbereich der Magnetresonanzvorrichtung, innerhalb dessen die Bedingungen für eine Erfassung von medizinischen Bilddaten, insbesondere von Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten aufweist. Insbesondere umfasst das Isozentrum den homogensten Magnetfeldbereich innerhalb der Magnetresonanzvorrichtung.

Für eine Positionierung des Patienten, insbesondere des zu untersuchenden Bereichs des Patienten, innerhalb des Patientenaufnahmebereich weist die Magnetresonanzvorrichtung die Patientenlagerungsvorrichtung auf. Die Patientenlagerungsvorrichtung ist zu einer Lagerung des Patienten ausgebildet. Die Patientenlagerungsvorrichtung weist bevorzugt einen bewegbaren Patiententisch auf, der insbesondere innerhalb des Patientenaufnahmebereichs der Magnetresonanzvorrichtung bewegbar ausgebildet ist. Bevorzugt ist hierbei der Patiententisch in Längsrichtung des Patientenaufnahmebereichs innerhalb des Patientenaufnahmebereichs bewegbar ausgebildet. Zudem ist der Patiententisch auch in vertikaler Richtung bewegbar ausgebildet, wobei der Patiententisch vor einem Einfahren in den Patientenaufnahmebereich in vertikaler Richtung auf eine Höhe von innerhalb des Patientenaufnahmebereichs angeordneten Führungsschiene eingestellt wird.

Für eine Einstellung einer Position des Patiententischs weist die Patientenlagerungsvorrichtung weiterhin eine Einstelleinheit. Die Einstelleinheit weist dabei eine Vertikaleinstelleinheit zur Einstellung einer vertikalen Position und/oder Höhe des Patiententischs auf. Zudem weist die Einstelleinheit eine Horizontaleinstelleinheit zu einer Einstellung einer horizontalen Position des Patiententischs, insbesondere in Längsrichtung des Patientenaufnahmebreichs, innerhalb des Patientenaufnahmebereichs auf.

Für eine Magnetresonanzuntersuchung wird der Patient zunächst auf dem Patiententisch der Patientenlagerungsvorrichtung positioniert. Anschließend wird der Patiententisch zusammen mit dem Patienten auf eine Höhe und/oder vertikale Position der Führungsschiene eingestellt. Danach wird der Patiententisch zusammen mit dem Patienten in den Patientenaufnahmebereich eingefahren, insbesondere in Längsrichtung des Patientenaufnahmebereichs, bis der zu untersuchende Bereich des Patienten innerhalb des Isozentrums positioniert ist.

Die Magnetresonanzvorrichtung weist weiterhin zumindest zwei unterschiedliche Führungsschieneneinheit auf. Die Führungsschieneneinheiten sind zu einer Führung und/oder Lagerung, insbesondere eine bewegbaren Führung und/oder Lagerung, des Patiententischs innerhalb des Patientenaufnahmebereichs ausgebildet. Die zumindest zwei unterschiedlichen Führungsschieneneinheiten unterscheiden sich hinsichtlich einer Führungshöhe und/oder einer vertikalen Position für den Patiententisch innerhalb des Patientenaufnahmebereichs. Bevorzugt sind die zumindest zwei unterschiedlichen Führungsschieneneinheiten baugleich hinsichtlich einer Befestigung und/oder Anordnung, insbesondere einer lösbaren Befestigung und/oder Anordnung, an der Umhausung ausgebildet.

Jede der Führungsschieneneinheiten weist zumindest ein Führungsschiene zur Führung des Patiententischs auf. Zudem können die Führungsschieneneinheiten auch zwei oder mehr Führungsschienen aufweisen, wobei die einzelnen Schienen unterschiedliche vertikale Positionen aufweisen. Die Führungsschieneneinheiten werden bevorzugt derart innerhalb des Patientenaufnahmebereichs an der Umhausung angeordnet, dass die Führungsschiene dem Patientenaufnahmebereich zugewandt ist. Befestigungselemente, die an der Führungsschieneneinheit für eine Positionierung und/oder Anordnung, insbesondere eine lösbare Befestigung, mit der Umhausung angeordnet sind, sind dagegen an einer der Umhausung zugewandten Seite der Führungsschieneneinheit angeordnet. Dabei können die Führungsschieneneinheiten jeweils zumindest zwei Führungsschienen auf, die bei einer Positionierung der Führungsschieneneinheit an der Umhausung an unterschiedlichen Seiten, insbesondere an gegenüberliegenden Seiten, der Umhausung positioniert und/oder angeordnet sind. Alternativ hierzu können die Führungsschieneneinheiten auch nur an einer Seite der Umhausung auswechselbar angeordnet sein, so dass für eine Führung des Patiententischs beidseitig eine Führungsschieneneinheit, wobei die beiden Führungsschieneneinheiten eine gleiche Führungshöhe zur Führung des Patiententischs innerhalb des Patientenaufnahmebereichs aufweisen.

Die auswechselbare Positionierung und/oder die auswechselbare Anordnung der Führungsschieneneinheiten an der Umhausung umfasst dabei, dass je nach gewünschter vertikaler Position des Patiententischs die entsprechende Führungsschieneneinheit an der Umhausung positioniert und/oder angeordnet wird. Führungsschieneneinheiten, die bereits innerhalb der Umhausung angeordnet sind, jedoch nicht die gewünschte vertikale Position für den Patiententisch aufweisen, werden vorher von der Umhausung demontiert und aus dem Patientenaufnahmebereich entfernt. Insbesondere können die Führungsschieneneinheiten abnehmbar an der Umhausung angeordnet und/oder befestigt werden. Die Führungseinheiten können dabei je nach Anwendungssituation, insbesondere Platzbedarf während einer Magnetresonanzuntersuchung, innerhalb des Patientenaufnahmebereichs an der Umhausung angeordnet und befestigt werden. Der Platzbedarf während einer Magnetresonanzuntersuchung kann dabei abhängig von einer Größe des Patienten und/oder einer Art der Untersuchung sein.

Zudem ist es auch denkbar, dass bei Verwendung eines freitragenden Patiententischs auch keine Führungsschieneneinheit innerhalb des Patientenaufnahmebereichs an der Umhausung positioniert wird, umso mehr Platz für ein Einführen des Patiententischs zusammen mit dem Patienten zu haben.

Die Erfindung weist den Vorteil auf, dass eine Position des Patiententischs während einer Magnetresonanzuntersuchung an einen Platzbedarf in vertikaler Richtung angepasst werden können. Insbesondere können bei einem erhöhten Platzbedarf für einer Magnetresonanzuntersuchung Führungsschieneneinheiten, die eine kleinere Führungshöhe als eine Standardführungshöhe des Patiententischs aufweisen, an der Umhausung angeordnet werden. Alternativ können auch bei beispielsweise Magnetresonanzuntersuchungen von Kleinkindern und/oder Säuglingen Führungsschienen, die eine größere Führungshöhe als eine Standardführungshöhe des Patiententischs aufweisen, an der Umhausung angeordnet werden. Dies ermöglicht auch eine optimale Positionierung des zu untersuchenden Bereichs des zu untersuchenden Patienten im Isozentrum der Magnetresonanzvorrichtung hinsichtlich einer Höhe und/oder einer vertikalen Ausrichtung. Damit einhergehend kann auch eine hohe Qualität in den erfassten Bilddaten erreicht werden.

Zudem können auch unterschiedliche Patientenlagerungsvorrichtungen, die unterschiedliche Führungseigenschaften innerhalb des Patientenaufnahmebereichs aufweisen, durch ein Anordnen der entsprechenden Führungsschieneneinheit für Magnetresonanzuntersuchungen an der gleichen Magnetresonanzvorrichtung verwendet werden. Insbesondere können dabei auch Patiententische mit unterschiedlichen Breiten für Magnetresonanzuntersuchungen verwendet werden.

Ein zusätzlicher Vorteil der Erfindung ist, dass durch ein Abnehmen der Führungsschieneneinheit eine einfache und schnelle Reinigung des Patientenaufnahmebereichs ermöglicht werden kann.

In einer vorteilhaften Weiterbildung der erfindungsgemäßen Magnetresonanzvorrichtung kann es vorgesehen sein, dass die Umhausung zumindest ein Befestigungselement aufweist für eine lösbare Anordnung einer der Führungsschieneneinheiten, wobei die Führungsschieneneinheiten jeweils ein zu dem Befestigungselement der Umhausung korrespondierendes Befestigungselement aufweisen. Die korrespondierenden Befestigungselemente sind bevorzugt derart ausgebildet, dass bei einer Verbindung der korrespondierenden Befestigungselemente eine Befestigung und/oder Anordnung, insbesondere eine lösbare Befestigung und/oder Anordnung, der jeweiligen Führungsschieneneinheit an der Umhausung erfolgt. Dabei kann das zumindest eine Befestigungselement der Führungsschieneneinheit eine Art Schiene aufweisen, beispielsweise eine T-förmige Schiene oder eine Schwalbenschwanz-förmige Schiene, die mit einer entsprechende Nut an der Umhausung zur Befestigung und/oder Anordnung der Führungsschieneneinheit korrespondiert. Alternativ hierzu kann das zumindest eine Befestigungselement einen Befestigungsbolzen, beispielsweise einen Pilzkopfbolzen, aufweisen, der mit eine entsprechenden Bohrung und/oder Aussparung der Umhausung zur Befestigung und/oder Anordnung der Führungsschieneneinheit korrespondiert. Zudem kann das zumindest eine Befestigungselement auch einen Schnellspannmechanismus umfassen.

Diese Ausgestaltung der Erdfindung ermöglicht eine einfache und schnelle Befestigung und/oder Anordnung der Führungsschieneneinheit an der Umhausung. Insbesondere können derart unterschiedliche Führungsschieneneinheiten, die insbesondere unterschiedliche Führungshöhen für den Patiententisch innerhalb des Patientenaufnahmebereichs aufweisen, einfach und schnell für eine Magnetresonanzuntersuchung ausgetauscht werden können.

In einer vorteilhaften Weiterbildung der erfindungsgemäßen Magnetresonanzvorrichtung kann es vorgesehen sein, dass die Umhausung zumindest zwei Befestigungselemente aufweist, die an gegenüberliegenden Seiten des von der Umhausung umgebenen Patientenaufnahmebereichs an der Umhausung angeordnet sind. Hierbei ist bevorzugt zumindest ein erstes Befestigungselement an einer ersten Seite der Umhausung und zumindest ein zweites Befestigungselement an einer zweiten Seite der Umhausung, insbesondere an einer der ersten Seite der Umhausung gegenüberliegenden Seite der Umhausung, angeordnet. Dabei kann die Umhausung auch mehr als ein erstes Befestigungselement und auch mehr als ein zweites Befestigungselement aufweisen. Die zumindest zwei Befestigungselemente sind bevorzugt baugleich ausgebildet. Die zumindest zwei Befestigungselemente können hierbei für eine Anordnung einer einzigen Führungsschieneneinheit ausgebildet sein, die damit an beiden Seiten, insbesondere den gegenüberliegenden Seiten, innerhalb des Patientenaufnahmebereichs befestigt werden kann. Alternativ hierzu können auch zwei Führungsschieneneinheiten mittels der zumindest zwei Befestigungselemente an den gegenüberliegenden Seiten an der Umhausung angeordnet, insbesondere lösbar und/oder auswechselbar angeordnet, werden. Die beiden gegenüberliegenden Seiten der Umhausung umfassen dabei eine linke Seite und eine rechte Seite der Umhausung für eine beidseitige Führung des Patiententischs innerhalb des Patientenaufnahmebereichs.

In einer vorteilhaften Weiterbildung der erfindungsgemäßen Magnetresonanzvorrichtung kann es vorgesehen sein, dass das zumindest eine Befestigungselement der Umhausung ein Formschlusselement aufweist. Bevorzugt weisen auch die Führungsschieneneinheiten jeweils zumindest ein als Formschlusselement ausgebildetes Befestigungselement auf, dass zu dem Formschlusselement der Umhausung kompatibel ausgebildet ist. Derart kann eine einfache und sichere Anordnung und/oder Befestigung der Führungsschieneneinheiten vorteilhaft erreicht werden.

In einer vorteilhaften Weiterbildung der erfindungsgemäßen Magnetresonanzvorrichtung kann es vorgesehen sein, dass das Formschlusselement eine sich in Längserstreckung des Patientenaufnahmebereichs erstreckende Nut umfasst. Die Nut kann dabei T-förmig oder auch Schwalbenschwanz-förmig ausgebildet sein und/oder weitere, dem Fachmann als sinnvoll erscheinende Formen und/oder Querschnitte aufweisen. Die Längserstreckung des Patientenaufnahmebereichs ist dabei parallel zur Einfahrrichtung des Patiententischs in den Patientenaufnahmebereich. Zudem ist die Längserstreckung des Patientenaufnahmebereichs parallel zur z-Richtung der Magnetresonanzvorrichtung. Vorteilhafterweise weist die Umhausung zwei als Nuten ausgebbildete Formschlusselemente auf, die an gegenüberliegenden und einander zugewandten Seiten der den Patientenaufnahmebereich umgebenden Umhausung angeordnet sind. Hierdurch kann vorteilhaft eine stabile und sichere Befestigung der zumindest einen Führungsschieneneinheit entlang der gesamten Umhausung des Patientenaufnahmebereichs erreicht werden. Insbesondere kann derart eine vorteilhafte Abstützung und/oder Gewichtsverteilung über die gesamte Länge des zumindest einen Befestigungselements, insbesondere der Nut, erreicht werden. Ein weiterer Vorteil ist, dass ein einfacher und insbesondere werkzeugloser Wechsel der unterschiedlichen Führungsschieneneinheiten an der Umhausung erfolgen kann.

In einer vorteilhaften Weiterbildung der erfindungsgemäßen Magnetresonanzvorrichtung kann es vorgesehen sein, dass die Umhausung zumindest zwei Befestigungselemente umfasst, die zu einer Befestigung von einer Führungsschieneneinheit ausgebildet sind. Bevorzugt sind die zumindest zwei Befestigungselemente nacheinander in Längsrichtung des Patientenaufnahmebereichs an der Umhausung angeordnet. Zudem kann die Umhausung auch mehr als zwei Befestigungselemente zur Befestigung und/oder Anordnung einer Führungsschieneneinheit aufweisen. Diese Ausgestaltung der Erfindung weist den Vorteil auf, dass eine stabile und sichere Anordnung und/oder Befestigung, insbesondere eine lösbare und/oder auswechselbare Anordnung, der Führungsschieneneinheit an der Umhausung erreicht werden.

In einer vorteilhaften Weiterbildung der erfindungsgemäßen Magnetresonanzvorrichtung kann es vorgesehen sein, dass die Scannereinheit eine Hochfrequenzantenneneinheit umfasst, wobei die zumindest zwei Befestigungselemente außerhalb eines Überdeckungsbereichs der Umhausung mit der Hochfrequenzantenneneinheit an der Umhausung angeordnet sind. Die Hochfrequenzantenneneinheit ist fest innerhalb der Scannereinheit, insbesondere der Magneteinheit, integriert. Bevorzugt ist die Hochfrequenzantenneneinheit zwischen der Gradientenspuleneinheit und der Umhausung innerhalb der Scannereinheit, insbesondere der Magneteinheit, angeordnet. Der Überdeckungsbereich der Hochfrequenzantenneneinheit mit der den Patientenaufnahmebereich umgebenden Umhausung erstreckt sich dabei in Längsrichtung des Patientenaufnahmebereichs und/oder in **z-**Richtung der Scannereinheit, insbesondere der Magneteinheit. Dabei umfasst der Überdeckungsbereich einen Bereich, bei dem die Hochfrequenzantenneneinheit und die Umhausung eine gleiche z-Koordinate aufweisen. Eine Anordnung der zumindest zwei Befestigungselemente außerhalb des Überdeckungsbereichs umfasst dabei eine Anordnung der zumindest zwei Befestigungselemente in einem Bereich, in dem die Hochfrequenzantenneneinheit kein Erstreckung in z-Richtung aufweist. Diese Ausgestaltung der Erfindung weist den Vorteil auf, dass eine unerwünschte Beeinträchtigung der Hochfrequenzantenneneinheit und/oder einer Magnetresonanzuntersuchung verhindert werden kann.

In einer vorteilhaften Weiterbildung der erfindungsgemäßen Magnetresonanzvorrichtung kann es vorgesehen sein, dass die zumindest zwei Befestigungselemente jeweils eine Bohrung und/oder eine Aussparung und/oder einen Stift umfassen. Bevorzugt ist die Bohrung und/oder Aussparung an einer dem Patientenaufnahmebereich zugewandten Seite der Umhausung angeordnet. Die Bohrung und/oder Aussparung ist zu einer Aufnahme eines Befestigungselements der Führungsschieneneinheiten ausgebildet. Die Bohrung und/oder Aussparung kann zudem einen Hohlraum umfassen, der zumindest teilweise von einem dem Patientenaufnahmebereich zugewandten Gehäuseseite verdeckt ist, beispielsweise zur Aufnahme eines Pilzkopf-Befestigungselements der Führungsschieneneinheiten. Der Stift kann dabei bolzenförmig und/oder zylinderförmig ausgebildet sein. Bevorzugt ist der Stift zu einer Anordnung und/oder Befestigung der Führungsschieneneinheiten an einer dem Patientenaufnahmebereich zugewandten Seite der Umhausung angeordnet. Zudem kann der Stift zu einer Anordnung und/oder Befestigung der Führungsschieneneinheiten auch abnehmbar an der Umhausung angeordnet sein. Diese Ausgestaltung der Erfindung weist den Vorteil auf, dass eine einfache Anordnung und/oder Befestigung einer Führungsschieneneinheit an der Umhausung ermöglicht werden kann. Insbesondere kann zudem eine schnelle und zeitsparende Befestigung und/oder Anordnung einer Führungsschieneneinheit an der Umhausung erreicht werden.

In einer vorteilhaften Weiterbildung der erfindungsgemäßen Magnetresonanzvorrichtung kann es vorgesehen sein, dass die unterschiedlichen Führungsschieneneinheiten jeweils eine Kodierung aufweisen, wobei die Kodierung eine Information bezüglich einer Führungshöhe der jeweiligen Führungsschieneneinheit umfasst. Die unterschiedliche Führungsschieneneinheiten weisen dabei bevorzugt unterschiedliche Kodierungen auf.

Die Kodierung kann bevorzugt eine mechanische Kodierung und/oder eine elektro-optische Kodierung aufweisen. Die mechanische Kodierung kann beispielsweise eine Steckverbindung und/oder einen Druckschalter und/oder weitere, dem Fachmann als sinnvoll erscheinende mechanische Kodierungen zwischen den Führungsschieneneinheiten und der Umhausung umfassen. Ein derartiger Druckschalter kann dabei mehrere Druckpunkte umfassen, wobei je nach Art der Führungsschieneneinheit eine unterschiedliche Kombination der mehreren Druckpunkte aktivierbar ist. Dabei weist die Führungsschieneneinheit beispielsweise einen entsprechenden kodierten Stecker auf, der in eine entsprechende Sensorbuchse, die beispielsweise an der Umhausung angeordnet ist, einführbar ist. Vorzugsweise wird bei einer Anordnung und/oder Befestigung der Führungsschieneneinheit an der Umhausung auch die entsprechende mechanische Kodierung aktiviert und/oder an die Umhausung übermittelt.

Alternativ oder zusätzlich kann die Kodierung auch eine elektro-optische Kodierung, beispielsweise einen Strichcode und/oder einen Barcode und/oder einen zweidimensionalen Barcode, umfassen. Zudem kann die elektro-optische Kodierung auch einen RFID-Code und/oder einen Reflektor und/oder weitere, dem Fachmann als sinnvoll erscheinende elektro-optische Kodierungen umfassen. Bevorzugt ist die Kodierung bei unterschiedlichen Führungsschieneneinheiten stets an einer gleichen Position an der Führungsschieneneinheit angeordnet, so dass eine einfache und schnelle Erfassung der Kodierung ermöglicht werden kann.

Diese Ausgestaltung der Erfindung ermöglicht eine einfache und schnelle Erfassung der an der Umhausung angeordneten Führungsschieneneinheit. Insbesondere ermöglicht diese Ausgestaltung der Erfindung eine automatische und/oder selbsttätige Erfassung der an der Umhausung angeordneten Führungsschieneneinheit. Derart kann auch einer Patiententischsteuereinheit, die zu einer Steuerung einer Bewegung des Patiententischs ausgebildet ist, eine exakte Höhe und/oder vertikale Position mitgeteilt werden, auf der Patiententisch angehoben werden muss, bevor er in den Patientenaufnahmebereich einfahren kann.

In einer vorteilhaften Weiterbildung der erfindungsgemäßen Magnetresonanzvorrichtung kann es vorgesehen sein, dass die Magnetresonanzvorrichtung zumindest ein Erfassungselement zur Erfassung der Kodierung der Führungsschieneneinheiten aufweist. Umfasst die Kodierung der Führungsschieneneinheiten eine mechanische Kodierung, umfasst das Erfassungselement bevorzugt ein mechanisches Erfassungselement zur Erfassung der mechanischen Kodierung, wie beispielsweise einen Stecker mit einem definierten Steckmuster usw. Umfasst die Kodierung der Führungsschieneneinheiten eine elektro-optische Kodierung, umfasst dabei das Erfassungselement bevorzugt eine elektrooptisches Sensoreinheit und/oder Leseeinheit. Dabei kann das Erfassungselement zur Erfassung einer elektro-optischen Kodierung zu einer kontaktlosen Erfassung der elektro-optischen Kodierung an der Führungsschieneneinheit ausgebildet sein. Hierdurch kann eine einfache und insbesondere automatische und/oder selbsttätige Erfassung der an der Umhausung angeordneten Führungsschieneneinheit erreicht werden. Derart kann auch einer Patiententischsteuereinheit, die zu einer Steuerung einer Bewegung des Patiententischs ausgebildet ist, eine exakte Höhe und/oder vertikale Position mitgeteilt werden, auf der Patiententisch angehoben werden muss, bevor er in den Patientenaufnahmebereich einfahren kann.

In einer vorteilhaften Weiterbildung der erfindungsgemäßen Magnetresonanzvorrichtung kann es vorgesehen sein, dass das zumindest eine Erfassungselement von der Patientenlagerungsvorrichtung, insbesondere dem Patiententisch, und/oder von der den Patientenaufnahmebereich umgebenden Umhausung umfasst ist. Derart kann eine besonders schnelle und direkte Erfassung der an der Umhausung angeordneten Führungsschieneneinheit bereitgestellt werden. Weist die Patientenlagerungsvorrichtung, insbesondere der Patiententisch, das zumindest eine Erfassungselement auf, können die erfassten Daten direkt an eine Patiententischsteuereinheit der Patientenlagerungsvorrichtung übertragen werden. Derart kann eine schnelle und direkte Einstellung einer Höhe und/oder einer vertikalen Position des Patiententischs vorteilhaft erreicht werden.

In einer vorteilhaften Weiterbildung der erfindungsgemäßen Magnetresonanzvorrichtung kann es vorgesehen sein, dass die Magnetresonanzvorrichtung eine Recheneinheit aufweist, wobei die Recheneinheit anhand der erfassten Kodierung durch das zumindest eine Erfassungselement eine einzustellende vertikale Position des Patiententischs bereitstellt.

Die Recheneinheit ist hierbei bevorzugt von der Patiententischsteuereinheit der Patientenlagerungsvorrichtung umfasst. Dabei umfasst die Recheneinheit zumindest ein Rechenmodul und/oder einen Prozessor und ist insbesondere dazu ausgebildet, computerlesbare Instruktionen auszuführen. Insbesondere umfasst die Recheneinheit eine Speichereinheit, wobei auf der Speichereinheit computerlesbare Informationen gespeichert sind, wobei die Recheneinheit dazu ausgebildet ist, die computerlesbaren Informationen von der Speichereinheit zu laden und die computerlesbaren Informationen auszuführen. Derart ist die erfindungsgemäße Recheneinheit dazu ausgebildet, anhand der erfassten Kodierung durch das zumindest eine Erfassungselement eine einzustellende vertikale Position des Patiententischs bereitzustellen. Die Komponenten der Recheneinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Die von dem zumindest einem Erfassungselement erfasste Kodierung der Führungsschieneneinheit, insbesondere die mit der erfasste Kodierung gekoppelte und/oder verbundene Positionsinformation der Führungsschieneneinheit, wird hierbei an die Recheneinheit übermittelt, beispielsweise mittels einer Datenübertragungseinheit. Die Datenübertragung der Datenübertragungseinheit kann dabei kabelgebunden sein oder auch kabellos und/oder drahtlos erfolgen. Anhand der Kodierung, insbesondere anhand der mit der erfassten Kodierung gekoppelte und/oder verbundene Positionsinformation der Führungsschieneneinheit, kann die Recheneinheit eine Höhe und/oder eine vertikale Position der Führungsschiene der Führungsschieneneinheit ermitteln und daraus die Position, insbesondere die vertikale Position, des Patiententischs ermitteln und bereitstellen. Die Position des Patiententischs wird von der Recheneinheit der Patiententischsteuereinheit an eine Einstelleinheit, insbesondere eine Vertikaleinstelleinheit, der Patientenlagerungsvorrichtung bereitgestellt. Diese Ausgestaltung der Erfindung weist den Vorteil auf, dass eine direkte und schnelle Einstellung einer Höhe und/oder einer vertikalen Position des Patiententischs bereitgestellt werden.

In einer vorteilhaften Weiterbildung der erfindungsgemäßen Magnetresonanzvorrichtung kann es vorgesehen sein, dass die Magnetresonanzvorrichtung zumindest eine Zusatzeinheit aufweist, die zumindest teilweise in eine der Führungsschieneneinheiten integriert ist. Eine derartige Zusatzeinheit kann beispielsweise eine Beleuchtungseinheit mit zumindest einem Beleuchtungselement und/oder Lichtabstrahlelement zur Beleuchtung des Patientenaufnahmebereichs umfassen. Bevorzugt weist hierbei die Führungsschieneneinheit eine optische Schnittstelle und/oder eine elektronische Schnittstelle auf zur Übertragung von Lichtsignalen und/oder von Steuersignalen zur Steuerung der innerhalb der Führungsschieneneinheit integrierten Beleuchtungseinheit. Alternativ oder zusätzlich kann die Zusatzeinheit auch eine Lüftungsöffnung für eine Luftzufuhr während einer Magnetresonanzuntersuchung umfassen. Bevorzugt weist hierbei die Führungsschieneneinheit einen Lüftungskanal und eine Lüftungsschnittstelle auf, wobei die Lüftungsschnittstelle mit einem Lüftungskanal einer von der Magneteinheit umfassten Lüftungseinheit koppelt. Alternativ oder zusätzlich kann die Zusatzeinheit auch zumindest ein Kommunikationselement einer Patientenkommunikationseinheit umfassen. Ein derartiges Kommunikationselement kann beispielsweise ein Patientenmikrofon und/oder ein Patientenlautsprecher umfassen. Bevorzugt weist hierzu die Führungsschieneneinheit eine Kommunikationsschnittstelle auf. Alternativ oder zusätzlich können weitere, dem Fachmann als sinnvoll erscheinende Zusatzeinheiten in die zumindest eine Führungsschieneneinheit integriert werden.

Diese Ausgestaltung der Erfindung ermöglicht eine vorteilhafte und insbesondere platzsparende Integration von weiteren Einheiten innerhalb des Patientenaufnahmebereichs. Insbesondere können derart platzsparend weitere Einheiten angeordnet werden, die während einer Magnetresonanzuntersuchung, beispielsweise für eine Sicherheit und/oder für ein Wohlbefinden des Patienten, erforderlich sind.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine erfindungsgemäße Magnetresonanzvorrichtung mit einer abnehmbaren Führungsschieneneinheit an einer Umhausung in einer schematischen Darstellung,
- Fig. 2: die Führungsschieneneinheit mit der Umhausung in einer Schnittdarstellung,
- Fig. 3: ein zweites Ausführungsbeispiel einer lösbaren Anordnung einer Führungsschieneneinheit an einer Umhausung in einer Schnittdarstellung,
- Fig. 4: ein weiteres Ausführungsbeispiel einer lösbaren Anordnung einer Führungsschieneneinheit an einer Umhausung in einer Schnittdarstellung,
- Fig. 5: eine Schnittdarstellung mit zwei unterschiedlichen Führungsschieneneinheiten an der Umhausung,
- Fig. 6: ein weiteres Ausführungsbeispiel einer lösbaren Anordnung einer Führungsschieneneinheit an einer Umhausung,
- Fig. 7: ein weiteres Ausführungsbeispiel einer lösbaren Anordnung einer Führungsschieneneinheit an der Umhau- sung, wobei die Führungsschieneneinheit eine Kodierung aufweist, und
- Fig. 8: ein weiters Ausführungsbeispiel einer lösbaren Anordnung einer Führungsschieneneinheit an der Umhausung, wobei die Führungsschieneneinheit eine Zusatzeinheit umfasst.

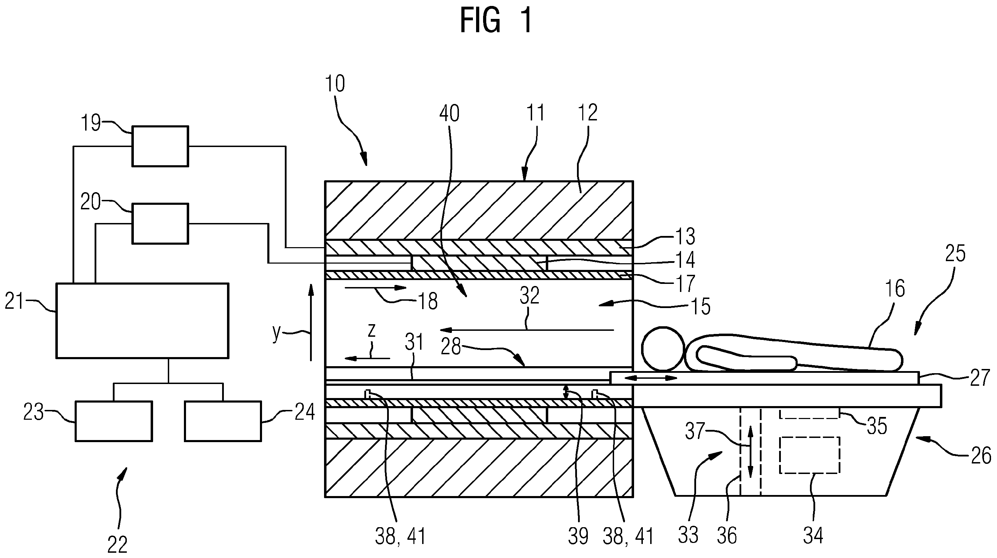

In der Fig. 1 ist eine Magnetresonanzvorrichtung 10 schematisch dargestellt. Die Magnetresonanzvorrichtung 10 umfasst eine von einer Magneteinheit 11 gebildeten Scannereinheit mit einem Grundmagneten 12, einer Gradientenspuleneinheit 13 und einer Hochfrequenzantenneneinheit 14. Zudem weist die Magnetresonanzvorrichtung 10 einen Patientenaufnahmebereich 15 auf zu einer Aufnahme Patienten 16 für eine Magnetresonanzuntersuchung. Der Patientenaufnahmebereich 15 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereich 15 jederzeit denkbar. Die Scannereinheit, insbesondere die Magneteinheit 11, weist weiterhin eine den Patientenaufnahmebereich 15 umgebende Umhausung 17 auf, wobei die Umhausung 17 den Patientenaufnahmebereich 15 zylinderförmig umgibt. Der Patientenaufnahmebereich 15 umfasst dabei denjenigen Bereich, der von der Umhausung 17 umgeben ist.

Der Grundmagnet 12 der Magneteinheit 11 ist zu einem Erzeugen eines starken und insbesondere konstanten Grundmagnetfelds 18 ausgebildet. Dabei kann der Grundmagnet 12 beispielsweise als supraleitender Grundmagnet 12 oder auch als Dauermagnet ausgebildet sein. Die Gradientenspuleneinheit 13 der Magneteinheit 11 ist zu einer Erzeugung von Magnetfeldgradienten, die für eine Ortskodierung während einer Bildgebung verwendet werden, ausgebildet. Die Gradientenspuleneinheit 13 wird mittels einer Gradientensteuereinheit 19 der Magnetresonanzvorrichtung 10 gesteuert. Die Hochfrequenzantenneneinheit 14 der Magneteinheit 11 ist zu einer Anregung einer Polarisation, die sich in dem von dem Grundmagneten 12 erzeugten Grundmagnetfeld 18 einstellt, ausgebildet. Die Hochfrequenzantenneneinheit 14 wird von einer Hochfrequenzantennensteuereinheit 20 der Magnetresonanzvorrichtung 10 gesteuert und strahlt hochfrequente Magnetresonanzsequenzen in den Aufnahmebereich 15 der Magnetresonanzvorrichtung 10 ein.

Zu einer Steuerung des Grundmagneten 12, der Gradientensteuereinheit 19 und zur Steuerung der Hochfrequenzantennensteuereinheit 20 weist die Magnetresonanzvorrichtung 10 eine Systemsteuereinheit 21 auf. Die Systemsteuereinheit 21 steuert zentral die Magnetresonanzvorrichtung 10, wie beispielsweise das Durchführen einer vorbestimmten bildgebenden Gradientenechosequenz. Zudem umfasst die Systemsteuereinheit 21 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 10 eine Benutzerschnittstelle 22, die mit der Systemsteuereinheit 21 verbunden ist. Steuerinformationen wie beispielsweise Bildgebungsparameter, sowie rekonstruierte Magnetresonanzbilder können auf einer Darstellungseinheit 23, beispielsweise auf zumindest einem Monitor, der Benutzerschnittstelle 22 für ein medizinisches Bedienpersonal angezeigt werden. Weiterhin weist die Benutzerschnittstelle 22 eine Eingabeeinheit 23 auf, mittels der Informationen und/oder Parameter während eines Messvorgangs von einem medizinischen Bedienpersonal eingegeben werden können.

Die dargestellte Magnetresonanzvorrichtung 10 kann selbstverständlich weitere Komponenten umfassen, die Magnetresonanzvorrichtungen 10 gewöhnlich aufweisen. Eine allgemeine Funktionsweise einer Magnetresonanzvorrichtung 10 ist zudem dem Fachmann bekannt, so dass auf eine detaillierte Beschreibung der weiteren Komponenten verzichtet wird.

Für eine Positionierung des Patienten 16, insbesondere eines zu untersuchenden Bereichs des Patienten 16, innerhalb des Patientenaufnahmebereich 15 weist die Magnetresonanzvorrichtung 10 eine Patientenlagerungsvorrichtung 25 auf. Die Patientenlagerungsvorrichtung 25 weist eine Basiseinheit 26 und einen bezüglich der Basiseinheit 26 bewegbaren Patiententisch 27 auf. Der Patiententisch 27 ist für eine Positionierung des Objekts und/oder des Patienten 16, insbesondere des zu untersuchenden Bereichs des Patienten 16, bewegbar innerhalb des Patientenaufnahmebereich 15 ausgebildet. Insbesondere ist hierbei der Patiententisch 27 in Richtung einer Längserstreckung des Patientenaufnahmebereich 15 und/oder in z-Richtung bewegbar gelagert. Innerhalb des Patientenaufnahmebereichs 15 ist zumindest eine Führungsschieneneinheit 28 der Magnetresonanzvorrichtung 10 angeordnet. Im vorliegenden Ausführungsbeispiel weist die Magnetresonanzvorrichtung 10 zwei Führungsschieneneinheiten 28 auf, die an gegenüberliegenden Seiten 29, 30 des Patientenaufnahmebereichs 15 an der Umhausung 17 angeordnet sind. Die beiden Führungsschieneneinheiten 29, 30 weisen jeweils zumindest eine Führungsschiene 31 auf, auf der der Patiententisch 27 für eine Bewegung in Längsrichtung 32 des Patientenaufnahmebereichs 15 innerhalb des Patientenaufnahmebereichs 15 geführt ist.

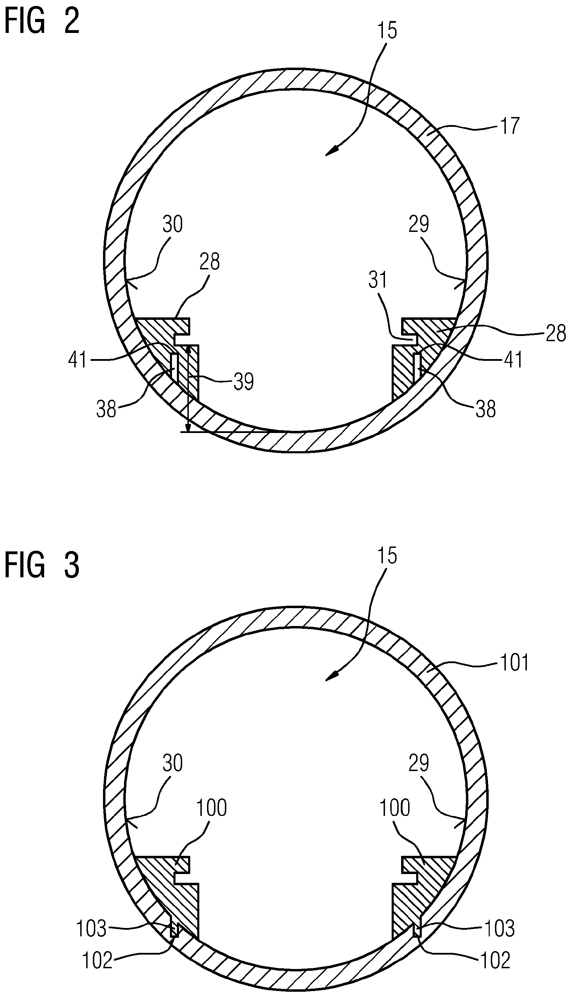

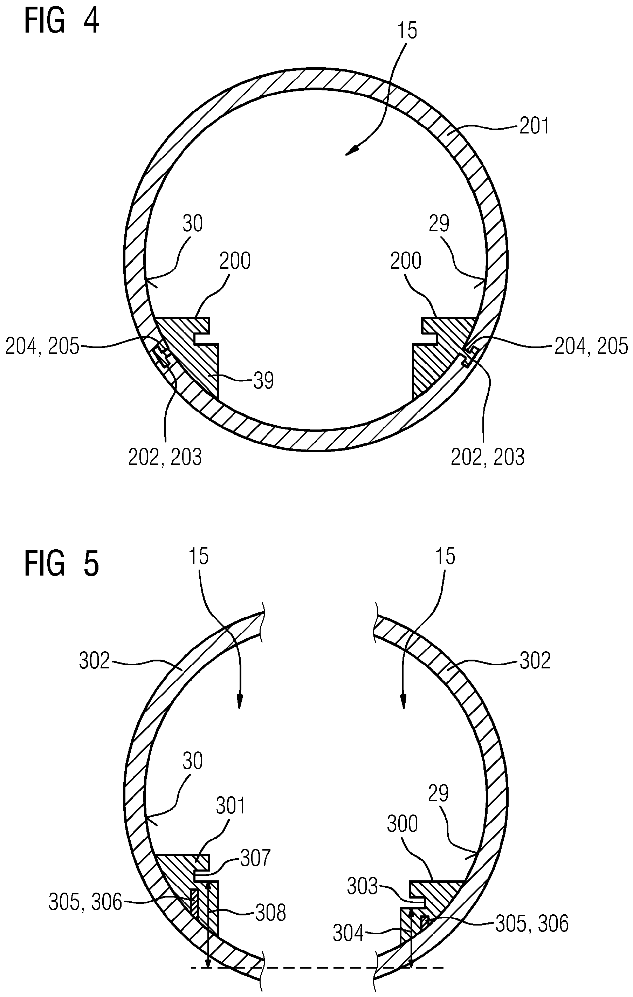

Die Magnetresonanzvorrichtung 10 weist zudem weitere Führungsschieneneinheiten 28, 300, 301 auf, wobei in Fig. 1 und 2 nur eine Führungsschieneneinheit 28 in einer an der Umhausung 17 angeordneten Position zu sehen ist. Die Führungsschieneneinheiten 28, 300, 301 sind auswechselbar an der Umhausung 17 angeordnet. Hinsichtlich einer Anordnung und/oder Befestigung, insbesondere einer auswechselbaren und/oder lösbaren Anordnung und/oder einer auswechselbaren und/oder lösbaren Befestigung, an der Umhausung 17 sind die unterschiedlichen Führungsschieneneinheiten 28, 300, 301 baugleich ausgebildet. Die Führungsschieneneinheiten 28, 300, 301 unterscheiden sich hierbei in einer Position der Führungsschiene 31 zur Führung des Patiententischs 27 und damit in einer Führungshöhe 39 (siehe Fig. 5).

Für eine Bewegung des Patiententischs 27 weist die Patientenlagerungsvorrichtung 25 eine Einstelleinheit 33 und eine Patiententischsteuereinheit 34 auf. Die Einstelleinheit 33 weist eine Horizontaleinstelleinheit 35 und eine Vertikaleinstelleinheit 36 auf. Die Horizontaleinstelleinheit 35 ist zu einem Einstellen einer Position des Patiententischs 27 in horizontaler Richtung, insbesondere in z-Richtung und/oder in Längsrichtung 32 des Patientenaufnahmebereichs 25 ausgebildet. Die Vertikaleinstelleinheit 36 ist zu einem Einstellen einer Höhe und/oder einer Position in vertikaler Richtung 37, insbesondere in y-Richtung des Patientenaufnahmebereichs 25, ausgebildet. Die Patiententischsteuereinheit 34 ist zu einer Steuerung der Einstelleinheit 33 ausgebildet und weist hierzu eine erforderliche Software und Hardware auf.

Für eine flexible Einstellung einer Höhe und/oder vertikalen Position des Patiententischs 27 innerhalb des Patientenaufnahmebereichs 15 weist die Umhausung 17 zumindest ein Befestigungselement 38 für eine lösbare Anordnung und/oder Befestigung der Führungsschieneneinheiten 28 auf. Im vorliegenden Ausführungsbeispiel sind zwei Führungsschieneneinheiten 29 an gegenüberliegenden Seiten der Umhausung 17 innerhalb des Patientenaufnahmebereichs 15 angeordnet (Fig. 2), wobei die beiden Führungsschieneneinheiten 28 hinsichtlich einer Führungshöhe 39 des Patiententischs 27 baugleich ausgebildet sind. Hierzu weist auch die Umhausung 17 an jeder der beiden gegenüberliegenden Seiten 29, 30 jeweils zumindest ein Befestigungselement auf zur Anordnung und/oder Befestigung der beiden Führungsschieneneinheiten 28.

Im vorliegenden Ausführungsbeispiel weist die Umhausung 17 zwei Befestigungselemente 38 zu einer Anordnung und/oder Befestigung einer Führungsschieneneinheit 28, insbesondere einer einzigen Führungsschieneneinheit 28, auf. Zur Befestigung der beiden Führungsschieneneinheiten 28 weist somit die Umhausung 17 vier Befestigungselemente 38 auf. In einer alternativen Ausgestaltung kann die Umhausung 17 auch mehr als zwei Befestigungselemente 38 zur Anordnung und/oder Befestigung einer Führungsschieneneinheit 28 aufweisen. Die vier Befestigungselemente 38 sind dabei jeweils außerhalb eines Überdeckungsbereichs 40 der Umhausung 17 mit der Hochfrequenzantenneneinheit 14 an der Umhausung 17 angeordnet.

Die einzelnen Befestigungselemente 38 weisen jeweils ein Formschlusselement auf (siehe Fig. 2). Die Formschlusselemente sind hierbei baugleich ausgebildet. Die einzelnen Befestigungselemente 38, insbesondere Formschlusselemente, umfassen dabei jeweils einen abnehmbaren Stift und/oder zylinderförmigen Bolzen, der an der Umhausung 17 befestigt ist und in den Patientenaufnahmebereich 15 hineinragt. Dabei sind der Befestigungselemente 38, insbesondere die Stifte, derart an der Umhausung 17 angeordnet, dass sie in vertikaler Richtung 37 und/oder in y-Richtung sich von der Umhausung 17 wegerstrecken und in den Patientenaufnahmebereich 15 hineinragen (Fig. 2).

Für eine Anordnung und/oder Befestigung der beiden Führungsschieneneinheiten 28 an der Umhausung 17 weisen die beiden Führungsschieneneinheiten 28 jeweils zwei Befestigungselemente 41 auf. Die Befestigungselemente 41 der beiden Führungsschieneneinheiten 28 sind dabei korrespondierend zu den Befestigungselementen 38 der Umhausung 17 ausgebildet, so dass bei einer mechanischen Verbindung der Befestigungselemente 41 der Führungsschieneneinheiten 28 mit den Befestigungselementen 38 der Umhausung 17 eine sichere Anordnung und/oder Befestigung der Führungsschieneneinheiten 28 an der Umhausung 17 erfolgt. Zudem ist eine Anzahl der Befestigungselemente 41 der Führungsschieneneinheiten 28 korrespondierend zu der Anzahl an Befestigungselementen 38 der Umhausung 17 ausgebildet. Die Befestigungselemente 41 der Führungsschieneneinheiten 28 sind dabei an einer der Umhausung 17 zugewandten Seite der Führungsschieneneinheiten 28 angeordnet. Dagegen ist die zumindest eine Führungsschiene 31 der Führungsschieneneinheit 28 an einer dem Patientenaufnahmebereich 15 zugewandten Seite der Führungsschieneneinheit 28 angeordnet.

Die einzelnen Befestigungselemente 41 der Führungsschieneneinheiten 28 umfassen dabei Aussparungen, die zur Aufnahme der Stifte und/oder zylinderförmige Bolzen der Umhausung 17 ausgebildet sind. Bei einer Befestigung der Führungsschieneneinheiten 28 an der Umhausung 17 ragen dabei die Stifte und/oder zylinderförmigen Bilden der Umhausung 17 in die Aussparungen der Führungsschieneneinheiten 28. Die Aussparungen können dabei auch einen im Querschnitt T-förmigen Bereich aufweisen, in dem beispielsweise Pilzkopf-förmige Stifte der Umhausung 17 für eine sichere Anordnung und/oder Befestigung der Führungsschieneneinheit 28 gesichert sind.

In Fig. 3 ist ein alternatives Ausführungsbeispiel einer Führungsschieneneinheit 100 und einer den Patientenaufnahmebereich 15 umgebenden Umhausung 101 einer Magnetresonanzvorrichtung 10 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in den Fig. 1 und 2, wobei bezüglich gleichbleibender Bauteile, Merkmale und Funktionen auf die Beschreibung des Ausführungsbeispiels in den Fig. 1 und 2 verwiesen wird.

In Fig. 3 ist eine den Patientenaufnahmebereich 15 umgebende Umhausung 101 der Magnetresonanzvorrichtung 10 dargestellt. Die Umhausung 101 weist zur lösbaren Anordnung und/oder Befestigung einer Führungsschieneneinheit 100 zumindest ein Befestigungselement 102 auf. Im vorliegenden Ausführungsbeispiel weist die Magnetresonanzvorrichtung 10 zwei Führungsschieneneinheiten 100 auf, die an gegenüberliegenden Seiten 29, 30 der den Patientenaufnahmebereich 15 umgebenden Umhausung 101 angeordnet sind. Die beiden Führungsschieneneinheiten 100 sind dabei auswechselbar an der Umhausung 101 angeordnet und/oder positioniert.

Dabei weist die Umhausung 101 zur lösbaren und/oder auswechselbaren Befestigung und/oder Anordnung jeweils einer Führungsschieneneinheit 100 zwei Befestigungselemente 102 auf. Die einzelnen Befestigungselemente 102 sind jeweils von Formschlusselementen gebildet, wobei die Formschlusselemente im vorliegenden Ausführungsbeispiel jeweils eine Bohrung und/oder Aussparung aufweisen. Die Befestigungselemente 102, insbesondere die Aussparungen und/oder Bohrungen, sind dabei jeweils außerhalb eines Überdeckungsbereichs 40 der Umhausung 101 mit der Hochfrequenzantenneneinheit 14 an der Umhausung 101 angeordnet.

Zudem weisen auch die beiden Führungsschieneneinheiten 100 jeweils Befestigungselemente 103 auf, die zu den Befestigungselementen 102 der Umhausung 101 kompatibel ausgebildet sind. Im vorliegenden Ausführungsbeispiel weisen die Befestigungselemente 103 der Führungsschieneneinheiten 100 Stifte und/oder Bolzen auf. Beispielsweise können die Befestigungselemente 103 der Führungsschieneneinheiten 100 Bolzen mit einem Pilzkopf umfassen, die in die Bohrungen und/oder Aussparungen der Umhausung 101 greifen und damit eine sichere Befestigung, insbesondere eine Verriegelung, der Führungsschieneneinheiten 100 an der Umhausung 101 ermöglichen. Zudem ist eine Anzahl der Befestigungselemente 103 der Führungsschieneneinheiten 100 korrespondierend zu der Anzahl an Befestigungselementen 102 der Umhausung 101 ausgebildet.

In Fig. 4 ist ein alternatives Ausführungsbeispiel einer Führungsschieneneinheit 200 und einer den Patientenaufnahmebereich 15 umgebenden Umhausung 201 einer Magnetresonanzvorrichtung 10 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu den Ausführungsbeispielen in den Fig. 1 bis 3, wobei bezüglich gleichbleibender Bauteile, Merkmale und Funktionen auf die Beschreibung der Ausführungsbeispiele in den Fig. 1 bis 3 verwiesen wird.

In Fig. 4 ist eine den Patientenaufnahmebereich 15 umgebende Umhausung 201 der Magnetresonanzvorrichtung 10 dargestellt. Die Umhausung 201 weist zur lösbaren Anordnung und/oder Befestigung einer Führungsschieneneinheit 200 ein Befestigungselement 202 auf. Im vorliegenden Ausführungsbeispiel weist die Magnetresonanzvorrichtung 10 zwei Führungsschieneneinheiten 200 auf, die an gegenüberliegenden Seiten 29, 30 der den Patientenaufnahmebereich 15 umgebenden Umhausung 201 angeordnet sind. Die beiden Führungsschieneneinheiten 200 sind dabei auswechselbar an der Umhausung 201 angeordnet und/oder positioniert.

Dabei weist die Umhausung 201 zur lösbaren Befestigung und/oder Anordnung jeweils einer Führungsschieneneinheit 200 ein einziges Befestigungselement 202 auf. Die einzelnen Befestigungselemente 202 sind jeweils von Formschlusselementen gebildet und umfassen eine sich in Längsrichtung 32 des Patientenaufnahmebereichs 15 erstreckende Nut 203. Die beiden Nuten 203 sind im vorliegenden Ausführungsbeispiel als T-Nut ausgebildet. Alternativ hierzu können die beiden Nuten 203 auch eine Schwalbenschanz-Nut und/oder ein weitere, dem Fachmann als sinnvoll erscheinende Form aufweisen.

Wie in Fig. 4 zu sehen ist, weisen auch die beiden Führungsschieneneinheiten 100 jeweils ein zu den von der Umhausung 201 umfassten Befestigungselementen 202 korrespondierendes Befestigungselement 204 auf. Im vorliegenden Ausführungsbeispiel weisen die beiden Führungsschieneneinheiten 200 jeweils eine T-förmige Schiene 205 auf, die in die T-förmige Nut an der Umhausung 201 einführbar ist.

In Fig. 5 ist ein alternatives Ausführungsbeispiel einer Führungsschieneneinheit 300, 301 und einer den Patientenaufnahmebereich 15 umgebenden Umhausung 302 einer Magnetresonanzvorrichtung 10 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu den Ausführungsbeispielen in den Fig. 1 bis 4, wobei bezüglich gleichbleibender Bauteile, Merkmale und Funktionen auf die Beschreibung der Ausführungsbeispiele in den Fig. 1 bis 4 verwiesen wird.

In Fig. 5 ist eine den Patientenaufnahmebereich 15 umgebende Umhausung 302 der Magnetresonanzvorrichtung 10 dargestellt. Im vorliegenden Ausführungsbeispiel sind beispielhaft zwei unterschiedliche Führungsschieneneinheiten 300, 301 an der Umhausung 302 angeordnet, um die unterschiedlichen Positionierungsmöglichkeiten mit mehreren Führungsschieneneinheiten 300, 301 aufzuzeigen. Für ein Einfahren des Patiententischs 27 innerhalb des Patientenaufnahmebereichs 15 und/oder ein Positionieren des Patiententischs 27 innerhalb des Patientenaufnahmebereichs 15 sind jedoch zwei baugleiche Führungsschieneneinheiten 300, 301 erforderlich, die an gegenüberliegenden Seiten 29, 30 der den Patientenaufnahmebereich 15 umgebenden Umhausung 302 angeordnet sind.

Die beiden Führungsschieneneinheiten 300, 301 sind dabei auswechselbar an der Umhausung 302 angeordnet und/oder positioniert. Die beiden unterschiedlichen Führungsschieneneinheiten 300, 301 sind dabei hinsichtlich einer Befestigung und/oder Anordnung an der Umhausung 302, insbesondere hinsichtlich einer lösbaren und/oder auswechselbaren Befestigung und/oder Anordnung an der Umhausung 302, baugleich ausgebildet. Die beiden unterschiedlichen Führungsschieneneinheiten 300, 301 sind hinsichtlich einer Anordnung einer Führungsschiene 303, 307 innerhalb des Patientenaufnahmebereichs 15 unterschiedlich ausgebildet. Dabei weisen die unterschiedliche ausgebildeten Führungsschieneneinheiten 300, 301 unterschiedliche Führungshöhen 304, 308 und/oder unterschiedliche vertikale Positionen zur Führung des Patiententischs 27 auf.

Für eine auswechselbare Anordnung und/oder eine auswechselbare Befestigung der beiden Führungsschieneneinheiten 300, 301 an der Umhausung 302 weisen im vorliegenden Ausführungsbeispiel die beiden Führungsschieneneinheiten 300, 301 jeweils zwei als Aussparung ausgebildete Befestigungselemente 305 auf, die zu einer Aufnahme von als Befestigungsbolzen ausgebildete Befestigungselemente 306 der Umhausung 302 ausgebildet sind, wie dies beispielhaft in den Fig. 1 und 2 beschrieben ist. In einer alternativen Ausgestaltung kann die Umhausung 302 auch als Aussparungen und/oder Bohrungen ausgebildete Befestigungselemente 306 aufweisen und die Führungsschieneneinheiten 300, 301 entsprechende als Stifte und/oder Bolzen ausgebildete Befestigungselemente 305, wie dies beispielhaft in Fig. 3 beschrieben ist. In einer alternativen Ausgestaltung kann die Umhausung 302 auch als Nuten ausgebildete Befestigungselemente 306 aufweisen und die Führungsschieneneinheiten 300, 301 ein entsprechendes als Schiene ausgebildetes Befestigungselement 305, wie dies beispielhaft in Fig. 4 beschrieben ist. Zudem sind weitere, dem Fachmann als sinnvoll erscheinende Ausgestaltungen der Befestigungselemente 305, 306 jederzeit denkbar.

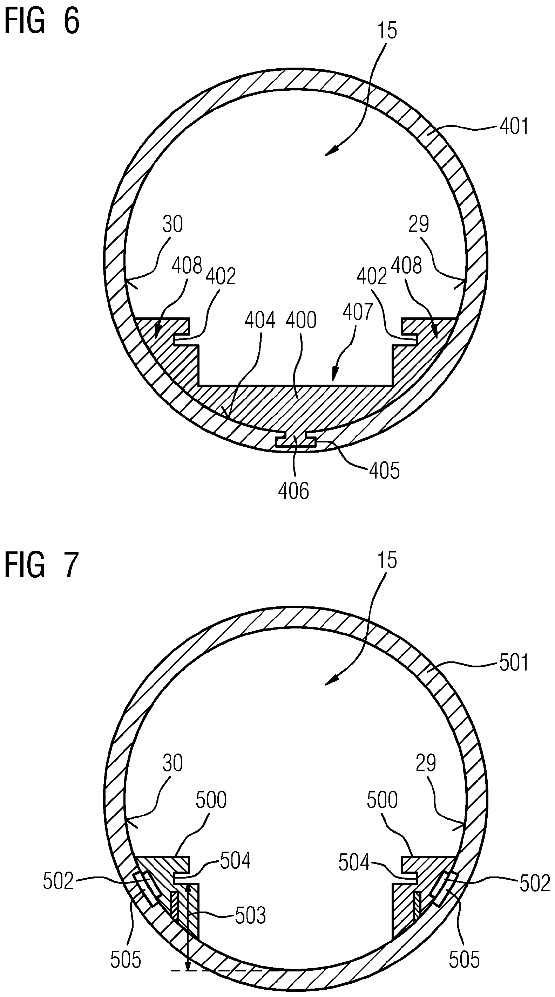

In Fig. 6 ist ein alternatives Ausführungsbeispiel einer Führungsschieneneinheit 400 und einer den Patientenaufnahmebereich 15 umgebenden Umhausung 401 einer Magnetresonanzvorrichtung 10 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu den Ausführungsbeispielen in den Fig. 1 bis 5, wobei bezüglich gleichbleibender Bauteile, Merkmale und Funktionen auf die Beschreibung der Ausführungsbeispiele in den Fig. 1 bis 5 verwiesen wird.

In Fig. 6 ist eine den Patientenaufnahmebereich 15 umgebende Umhausung 401 der Magnetresonanzvorrichtung 10 dargestellt. Im vorliegenden Ausführungsbeispiel weist die Magnetresonanzvorrichtung 10 eine einzige Führungsschieneneinheit 400 mit zwei Führungsschienen 402 auf. Die Führungsschieneneinheit 400 ist dabei auswechselbar und/oder lösbar an der Umhausung 401 angeordnet und/oder befestigt.

Zur lösbaren und/oder auswechselbaren Anordnung der Führungsschieneneinheit 400 weist die Umhausung 401 ein Befestigungselement 403 auf. Das zumindest eine Befestigungselement 403 ist dabei an einer Bodenfläche 404 der den Patientenaufnahmebereich 15 umgebenden Umhausung 401 angeordnet. Das Befestigungselement 405 der Umhausung 405 ist bespielhaft als Nut ausgebildet, wobei ein dazu korrespondierendes Befestigungselement 406 der Führungsschieneneinheit 400 als Schiene ausgebildet ist, wie dies insbesondere in Fig. 4 beschrieben ist. In einer alternativen Ausgestaltung können die Befestigungselemente 405, 406 auch als Aussparungen und/oder Bohrungen oder als Stifte und/oder Bolzen ausgebildet sein, wie dies in Fig. 1 und 2 oder in Fig. 3 beschrieben ist.

Die Führungsschieneneinheit 400 weist zudem einen u-förmigen Querschnitt auf, wobei in einem zentralen und/oder mittleren Bereich 407 das zumindest eine Befestigungselement 406 angeordnet ist. An Seitenbereichen 408, die sich von dem zentralen Bereich 407 nach oben wegerstrecken, sind die Führungsschienen 402 angeordnet. Die Seitenbereiche 408 liegen dabei jeweils an gegenüberliegenden Seiten 29, 30 der Umhausung 401 an.

In Fig. 7 ist ein alternatives Ausführungsbeispiel einer Führungsschieneneinheit 500 und einer den Patientenaufnahmebereich 15 umgebenden Umhausung 501 einer Magnetresonanzvorrichtung 10 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu den Ausführungsbeispielen in den Fig. 1 bis 6, wobei bezüglich gleichbleibender Bauteile, Merkmale und Funktionen auf die Beschreibung der Ausführungsbeispiele in den Fig. 1 bis 6 verwiesen wird.

In Fig. 7 ist eine den Patientenaufnahmebereich 15 umgebende Umhausung 501 der Magnetresonanzvorrichtung 10 dargestellt.

Innerhalb des Patientenaufnahmebereichs 15 sind zwei Führungsschieneneinheiten 500 an der Umhausung 501 auswechselbar angeordnet und/oder befestigt. Eine Ausgestaltung der Führungsschieneneinheiten 500 und eine lösbare und/oder auswechselbare Befestigung und/oder Anordnung an der Umhausung 501 entspricht dabei den Ausführungen zu den Fig. 1 und 2, auf die hiermit verwiesen wird. In einer alternativen Ausgestaltung kann die lösbare und/oder auswechselbare Befestigung und/oder lösbare Anordnung der Führungsschieneneinheiten 500 an der Umhausung 501 auch mittels Nuten und Schienen und/oder mittels Aussparungen und/oder mittels weiterer, dem Fachmann als sinnvoll erscheinender Befestigungselemente erfolgen.

Die beiden Führungsschieneneinheiten 500 im vorliegenden Ausführungsbeispiel weisen jeweils eine Kodierung 502 auf, die ein Erfassen der zumindest einen Führungsschieneneinheit 500 an der an der Umhausung 501 angeordneten Position ermöglicht. Mittels der Kodierung 502 kann dabei eine Information bezüglich einer vertikalen Position und/oder einer Führungshöhe 503 einer Führungsschiene 504 der Führungsschieneneinheit 500 bestimmt und/oder erfasst werden. Zur Erfassung der Kodierung 502 der Führungsschieneneinheiten 500 weist die Magnetresonanzvorrichtung 10 zumindest ein Erfassungselement 505 auf. Im vorliegenden Ausführungsbeispiel weist die Magnetresonanzvorrichtung 10 zwei Erfassungselemente 505 auf, die an der den Patientenaufnahmebereich 15 umgebenden Umhausung 501 angeordnet sind. Alternativ hierzu können die Erfassungselemente 505 auch an dem Patiententisch 27 der Patientenlagerungsvorrichtung 25 angeordnet sein.

Im vorliegenden Ausführungsbeispiel umfasst die Kodierung 502 eine elektro-optische Kodierung 502, insbesondere einen Barcode. Zudem umfassen die Erfassungselemente 505 ein entsprechendes Sensorelement und/oder Leseelement zum Erfassen der elektro-optischen Kodierung 502, insbesondere des Barcodes. In einer alternativen Ausgestaltung der Kodierung 502 kann diese auch eine mechanische Kodierung umfassen und das das Erfassungselement 505 ein mechanisches Erfassungselement, wie beispielsweise eine Steckverbindung und/oder einen Druckschalter.

Die von den Erfassungselementen 505 erfassten Informationen werden über eine nicht näher dargestellte Datenübertragungseinheit an die Patiententischsteuereinheit 34 übertragen. Die Patiententischsteuereinheit 34 umfasst dabei ein Rechenmodul und/oder eine Recheneinheit mit einem Prozessor auf, der anhand der erfassten Kodierung 502 und/oder anhand der erfassten Informationen der Kodierung 502 eine einzustellende Position des Patiententischs 27 bereitstellt. Die einzustellende Position umfasst dabei eine vertikale Position und/oder eine Höhe des Patiententischs 27, in die dieser gebracht werden muss, um ein Einfahren des Patiententischs 27 in den Patientenaufnahmebereichs 15 mithilfe der Führungsschienen 504 zu ermöglichen. Die einzustellende Position des Patiententischs 27 wird hierbei von der Patiententischsteuereinheit 34 der Einstelleinheit 33, insbesondere der Vertikaleinstelleinheit 36, bereitgestellt.

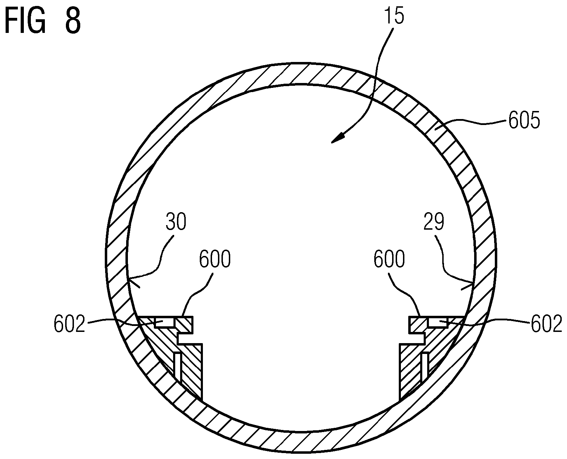

In Fig. 8 ist ein alternatives Ausführungsbeispiel einer Führungsschieneneinheit 600 und einer den Patientenaufnahmebereich 15 umgebenden Umhausung 601 einer Magnetresonanzvorrichtung 10 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu den Ausführungsbeispielen in den Fig. 1 bis 7, wobei bezüglich gleichbleibender Bauteile, Merkmale und Funktionen auf die Beschreibung der Ausführungsbeispiele in den Fig. 1 bis 7 verwiesen wird.

In Fig. 8 ist eine den Patientenaufnahmebereich 15 umgebende Umhausung 601 der Magnetresonanzvorrichtung 10 dargestellt. Innerhalb des Patientenaufnahmebereichs 15 sind zwei Führungsschieneneinheiten 600 an der Umhausung 601 auswechselbar angeordnet und/oder befestigt. Eine Ausgestaltung der Führungsschieneneinheiten 600 und eine lösbare und/oder auswechselbare Befestigung und/oder Anordnung an der Umhausung 601 entspricht dabei den Ausführungen zu den Fig. 1 und 2, auf die hiermit verwiesen wird. In einer alternativen Ausgestaltung kann die lösbare und/oder auswechselbare Befestigung und/oder lösbare Anordnung der Führungsschieneneinheiten 500 an der Umhausung 601 auch mittels Nuten und Schienen und/oder mittels Aussparungen und/oder mittels weiterer, dem Fachmann als sinnvoll erscheinender Befestigungselemente erfolgen.

Die Führungsschieneneinheiten 600 im vorliegenden Ausführungsbeispiel weisen eine Zusatzeinheit 602 auf, die in die Führungsschieneneinheiten 600 integriert ist. Die Zusatzeinheit 602 kann beispielsweise eine Beleuchtungseinheit mit zumindest einem Beleuchtungselement und/oder Lichtabstrahlelement zur Beleuchtung des Patientenaufnahmebereichs 15 umfassen. Alternativ oder zusätzlich kann die Zusatzeinheit 602 auch eine Lüftungsöffnung für eine Luftzufuhr innerhalb des Patientenaufnahmebereichs 15 während einer Magnetresonanzuntersuchung umfassen. Alternativ oder zusätzlich kann die Zusatzeinheit 602 auch zumindest ein Kommunikationselement einer Patientenkommunikationseinheit umfassen. Bevorzugt weist die Magnetresonanzvorrichtung 10 eine entsprechende, nicht näher dargestellte, Schnittstelle zwischen der Führungsschieneneinheit 600 und der Umhausung 601 auf, um eine Funktionsweise der Zusatzeinheit 602 zu ermöglichen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzvorrichtung mit einer Scannereinheit, einen von der Scannereinheit zumindest teilweise umgebenen Patientenaufnahmebereich, wobei die Scannereinheit eine den Patientenaufnahmebereich zumindest teilweise umgebende Umhausung aufweist, einer Patientenlagerungsvorrichtung mit einem bewegbaren Patiententisch, wobei der Patiententisch in den Patientenaufnahmebereich einfahrbar ausgebildet ist, **gekennzeichnet durch** zumindest zwei unterschiedlich ausgebildete Führungsschieneneinheiten, wobei die zumindest zwei unterschiedlich ausgebildeten Führungsschieneneinheiten unterschiedliche Führungshöhen für eine Führung des Patiententischs innerhalb des Patientenaufnahmebereichs aufweisen und wobei die zumindest zwei unterschiedlich ausgebildeten Führungsschieneneinheiten an der Umhausung auswechselbar positionierbar sind.

2. Magnetresonanzvorrichtung nach Anspruch 1,
**dadurch gekennzeichnet, dass** die Umhausung zumindest ein Befestigungselement aufweist für eine lösbare Anordnung einer der Führungsschieneneinheiten, wobei die Führungsschieneneinheiten jeweils ein zu dem Befestigungselement der Umhausung korrespondierendes Befestigungselement aufweisen.

3. Magnetresonanzvorrichtung nach einem der vorhergehenden Ansprüche,
**dadurch gekennzeichnet, dass** die Umhausung zumindest zwei Befestigungselemente aufweist, die an gegenüberliegenden Seiten des von der Umhausung umgebenen Patientenaufnahmebereichs an der Umhausung angeordnet sind.

4. Magnetresonanzvorrichtung nach einem der Ansprüche 2 oder 3,
**dadurch gekennzeichnet, dass** das zumindest eine Befestigungselement der Umhausung ein Formschlusselement aufweist.

5. Magnetresonanzvorrichtung nach Anspruch 4,
**dadurch gekennzeichnet, dass** das Formschlusselement eine sich in Längserstreckung des Patientenaufnahmebereichs sich erstreckende Nut umfasst.

6. Magnetresonanzvorrichtung nach einem der vorhergehenden Ansprüche,
**dadurch gekennzeichnet, dass** die Umhausung zumindest zwei Befestigungselemente umfasst, die zu einer Befestigung von einer Führungsschieneneinheit ausgebildet sind.

7. Magnetresonanzvorrichtung nach Anspruch 6,
**dadurch gekennzeichnet, dass** die Scannereinheit eine Hochfrequenzantenneneinheit umfasst, wobei die zumindest zwei Befestigungselemente außerhalb eines Überdeckungsbereichs der Umhausung mit der Hochfrequenzantenneneinheit an der Umhausung angeordnet sind.

8. Magnetresonanzvorrichtung nach einem der Ansprüche 6 oder 7,
**dadurch gekennzeichnet, dass** die zumindest zwei Befestigungselement jeweils eine Bohrungen und/oder Aussparungen und/oder einen Stift umfassen.

9. Magnetresonanzvorrichtung nach einem der vorhergehenden Ansprüche,
**dadurch gekennzeichnet, dass** die unterschiedlichen Führungsschieneneinheiten jeweils eine Kodierung aufweisen, wobei die Kodierung eine Information bezüglich einer Führungshöhe der jeweiligen Führungsschieneneinheit umfasst.

10. Magnetresonanzvorrichtung nach Anspruch 9,
durch zumindest einem Erfassungselement zur Erfassung der Kodierung der Führungsschieneneinheiten.

11. Magnetresonanzvorrichtung nach Anspruch 10,
**dadurch gekennzeichnet, dass** das zumindest eine Erfassungselement von der Patientenlagerungsvorrichtung, insbesondere von dem Patiententisch, und/oder von den Patientenaufnahmebereich umgebenden Umhausung umfasst ist.

12. Magnetresonanzvorrichtung nach einem der Ansprüche 9 bis 11,
**gekennzeichnet durch** eine Recheneinheit, die anhand der erfassten Kodierung durch das zumindest eine Erfassungselement eine einzustellende vertikale Position des Patiententischs bereitstellt.

13. Magnetresonanzvorrichtung nach einem der vorhergehenden Ansprüche,
**gekennzeichnet durch** zumindest eine Zusatzeinheit, die zumindest teilweise in eine der Führungsschieneneinheiten integriert ist.

## Claims

1. Magnetic resonance apparatus with a scanner unit, a patient accommodation region surrounded at least in part by the scanner unit, wherein the scanner unit has an enclosure at least in part surrounding the patient accommodation region, and a patient positioning apparatus with a movable patient table, wherein the patient table is configured to be advanceable into the patient accommodation region, **characterised by** at least two differently configured guide rail units, wherein the at least two differently configured guide rail units have different guide heights for guiding the patient table within the patient accommodation region and wherein the at least two differently configured guide rail units are interchangeably positionable on the enclosure.

2. Magnetic resonance apparatus according to claim 1, **characterised in that** the enclosure has at least one securing element for detachable arrangement of one of the guide rail units, wherein the guide rail units in each case has a securing element corresponding to the securing element of the enclosure.

3. Magnetic resonance apparatus according to one of the preceding claims,
**characterised in that** the enclosure has at least two securing elements which are arranged on the enclosure on opposing sides of the patient accommodation region surrounded by the enclosure.

4. Magnetic resonance apparatus according to one of claims 2 or 3,
**characterised in that** the at least one securing element of the enclosure has a positive-locking element.

5. Magnetic resonance apparatus according to claim 4,
**characterised in that** the positive-locking element comprises a groove extending in the longitudinal extent of the patient accommodation region.

6. Magnetic resonance apparatus according to one of the preceding claims,
**characterised in that** the enclosure comprises at least two securing elements which are configured to secure a guide rail unit.

7. Magnetic resonance apparatus according to claim 6,
**characterised in that** the scanner unit comprises a radio-frequency antenna unit, wherein the at least two securing elements in each case are arranged on the enclosure outside an overlap region of the enclosure with the radio-frequency antenna unit.

8. Magnetic resonance apparatus according to one of claims 6 or 7,
**characterised in that** the at least two securing elements in each case comprise a hole and/or an opening and/or a peg.

9. Magnetic resonance apparatus according to one of the preceding claims,
**characterised in that** the different guide rail units in each case have an encoding, wherein the encoding comprises information relating to a guide height of the respective guide rail unit.

10. Magnetic resonance apparatus according to claim 9,
**characterised by** at least one detection element for detecting the encoding of the guide rail units.

11. Magnetic resonance apparatus according to claim 10, **characterised in that** the at least one detection element is encompassed by the patient positioning apparatus, in particular by the patient table, and/or by the enclosure surrounding the patient accommodation region.

12. Magnetic resonance apparatus according to one of claims 9 to 11,
**characterised by** a computing unit which, using the encoding detected by the at least one detection element, provides a vertical position, which is to be established, of the patient table.

13. Magnetic resonance apparatus according to one of the preceding claims,
**characterised by** at least one auxiliary unit which is integrated at least in part into one of the guide rail units.

## Revendications

1. Installation de résonnance magnétique comprenant une unité de scanner, une zone de réception de patient entourée au moins en partie de l'unité de scanner, dans laquelle l'unité de scanner a une enceinte entourant au moins en partie la zone de réception d'un patient, une installation de couchette de patient contenant une table de patient mobile, dans laquelle la table de patient est constituée pour pouvoir rentrer dans la zone de réception d'un patient,
**caractérisée par** au moins deux unités de rail de guidage constituées différemment, dans laquelle les au moins deux unités de rail de guidage constituées différemment ont des niveaux de guidage différents pour un guidage de la table de patient dans la zone de réception d'un patient et dans laquelle les au moins deux unités de rail de guidage constituées différemment peuvent être mises en position avec possibilité de changement sur l'enceinte.

2. Installation de résonnance magnétique suivant la revendication 1,
**caractérisée en ce que** l'enceinte a au moins un élément de fixation pour un montage amovible de l'une des unités de rail de guidage, dans laquelle les unités de rail de guidage ont respectivement un élément de fixation correspondant à l'élément de fixation de l'enceinte.

3. Installation de résonnance magnétique suivant l'une des revendications précédentes,
**caractérisée en ce que** l'enceinte a deux éléments de fixation, qui sont montés sur l'enceinte sur des côtés opposés de la zone de réception d'un patient entourée de l'enceinte.

4. Installation de résonnance magnétique suivant l'une des revendications 2 ou 3,
**caractérisée en ce qu'**au moins un élément de fixation de l'enceinte a un élément à complémentarité de forme.

5. Installation de résonnance magnétique suivant la revendication 4,
**caractérisée en ce que** l'élément à complémentarité de forme comprend une rainure s'étendant dans l'étendue longitudinale de la zone de réception d'un patient.

6. Installation de résonnance magnétique suivant l'une des revendications précédentes,
**caractérisée en ce que** l'enceinte comprend au moins deux éléments de fixation, qui sont constitués pour une fixation d'une unité de rail de guidage.

7. Installation de résonnance magnétique suivant la revendication 6,
**caractérisée en ce que** l'unité de scanner comprend une unité d'antenne de haute fréquence, dans laquelle les au moins deux éléments de fixation sont montés sur l'enceinte à l'extérieur d'une zone de recouvrement de l'enceinte avec l'unité d'antenne de haute fréquence.

8. Installation de résonnance magnétique suivant l'une des revendications 6 ou 7,
**caractérisée en ce que** les au moins deux éléments de fixation comprennent chacun un alésage et/ou un creux et/ou une broche.

9. Installation de résonnance magnétique suivant l'une des revendications précédentes,
**caractérisée en ce que** les éléments de rail de guidage différents ont chacun un codage, dans laquelle le codage prend une information se rapportant à un niveau de guidage de l'unité de rail de guidage respective.

10. Installation de résonnance magnétique suivant la revendication 9,
**caractérisée par** au moins un élément de saisie pour la saisie du codage des unités de rail de guidage.

11. Installation de résonnance magnétique suivant la revendication 10,
**caractérisée en ce qu'**au moins un élément de saisie est compris par l'installation de couchette de patient, en particulier par la table de patient et/ou par l'enceinte entourant la zone de réception d'un patient.

12. Installation de résonnance magnétique suivant l'une des revendications 9 à 11,
**caractérisée par** une unité informatique, qui, à l'aide de la saisie d'un codage par le au moins un élément de saisie, donne une position verticale à régler de la table de patient.

13. Installation de résonnance magnétique suivant l'une des revendications précédentes,
**caractérisée par** au moins une unité supplémentaire, qui est intégrée au moins en partie dans l'une des unités de rail de guidage.
